# EUROPÄISCHE PATENTANMELDUNG

(11) **EP 3 450 537 A1**
(43) Veröffentlichungstag der Anmeldung: **06.03.2019**
(21) Anmeldenummer: 18190599.3
(22) Anmeldetag: 23.08.2018
(51) Int. Cl.: C12M 1/107, C12M 1/06, C12M 1/21, C12M 1/34, C12M 1/36

(54) **EINRICHTUNG UND VERFAHREN ZUR ERFASSUNG EINES ZUSTANDSPARAMETERS VON GÄRSUBSTRAT IN EINEM REAKTORBEHÄLTER**

(30) Priorität: 29.08.2017 DE 102017119785
(71) Anmelder: Räss, Martin, 94362 Neukirchen (DE); Schiessl, Ralf, 94362 Neukirchen (DE); Dallinger, Markus, 84329 Wurmannsquick (DE)
(72) Erfinder: Räss, Martin, 94362 Neukirchen (DE); Schiessl, Ralf, 94362 Neukirchen (DE); Dallinger, Markus, 84329 Wurmannsquick (DE)
(74) Vertreter: Benninger, Johannes

(57) **Zusammenfassung**

Es sind eine Einrichtung (30) sowie ein Verfahren zur Erfassung eines Zustandsparameters eines gärfähigen Materials (8) oder Substrats (8) in einem Reaktorbehälter (4) offenbart. Die genannte Einrichtung (30) umfasst dabei mindestens einen Aktor (14) welcher Substratumwälzungen herbeiführt, wobei mindestens eine unterhalb eines Gärsubstratpegels (8') angeordnete Kraftmessvorrichtung (16) Bewegungskräfte des Gärsubstrats (8) ermittelt, welche durch die Substratumwälzung auf die Kraftmessvorrichtung (16) einwirken.

Es ist vorgesehen, dass die Kraftmessvorrichtung (16) mit mindestens einer Datenübertragungseinrichtung (26) verbunden ist, welche mittels Drehzahl und/oder Stromverbrauch von mindestens einem Aktor (14) auf Basis der ermittelten Bewegungskräfte einen Zustandsparameter des Gärsubstrats (8) berechnet.

## Beschreibung

Die vorliegende Erfindung betrifft eine Einrichtung zur Erfassung eines Zustandsparameters von Gärsubstrat in einem zumindest teilweise mit gärfähigem Material beladenen oder beladbaren Reaktorbehälter mit den Merkmalen des unabhängigen Anspruchs 1. Die Erfindung betrifft zudem ein Verfahren zur Erfassung mindestens eines Zustandsparameters eines gärfähigen Materials oder Substrats in einem Reaktorbehälter mit den Merkmalen des unabhängigen Verfahrensanspruchs.

Die Gärprozesse innerhalb der Gärreaktorbehälter von Biogasanlagen sind für die unterschiedlichsten zu vergärenden Substrate in groben Zügen bekannt und zumindest teilweise von außen steuerbar, so etwa mittels Rührwerken, durch Zugabe von die Gärung unterstützenden Substanzen, durch Zugabe von gärfähigem Substrat, durch Zugabe oder Entnahme von Flüssigkeit, durch Entnahme von durch die Gärung entstandenem Gas, durch Variation und/oder Steuerung der Temperaturen im Gärbehälter, durch Einspeisung und/oder Entnahme von Wasser, von Sauerstoff oder durch weitere derartige oder andere Maßnahmen.

Dennoch ist es zur besseren Überwachung der im Gärreaktor stattfindenden Prozesse in vielen Fällen wünschenswert, mittels unterhalb des Flüssigkeitsspiegels des Gärsubstrats angeordneter Sensoren die Vorgänge innerhalb des Gärsubstrats genauer überwachen und/oder den Zustand des Substrats exakter beurteilen zu können, was insbesondere mit Hilfe von im Reaktorbehälter angeordneten Sensorsystemen möglich ist. Mittels des Einsatzes solcher Sensorsysteme sind nicht nur Fernüberwachungen in Echtzeit, d.h. ohne zwischenzeitliche Speicherung und späteres Auslesen der Sensorwerte, ermöglicht, sondern sie ermöglichen es je nach Signal- und Datenverarbeitung darüber hinaus, eine Aufzeichnung und Speicherung der erfassten Sensorwerte vorzunehmen, um etwa eine ständige oder in zyklischen Abständen erfolgende Analyse der Gärprozesse durchzuführen.

Damit die Gärprozesse in einem Biogasreaktor einwandfrei ablaufen können, sind oftmals aufwendige Kontrollen nötig, um insbesondere Schwimmschichten zu vermeiden. Solche unerwünschten Schwimmschichten sind auf dem Substrat bzw. auf der Substratoberfläche aufschwimmende Feststoffansammlungen, welche die Gasdiffusion vom Substrat in die Gasphase inhibieren und folglich den Gasertrag der gesamten Anlage erheblich beeinträchtigen können. Sehr kompakte Schwimmschichten können von den fermenterinternen Rührwerken oftmals nicht mehr beseitigt werden und müssen ggf. manuell entfernt werden, was zu erheblichen Zusatzkosten für die Anlagenbetreiber führen kann.

Hinsichtlich der Erfassung von Zustandsparametern von gärenden Substratbeladungen in Biogasreaktoren sei auf das Dokument EP 2553082 B1 verwiesen, welches ein Verfahren und eine Vorrichtung zur Herstellung von Biogas sowie zur Regelung von Gärprozessen offenbart.

Angesichts der im Stand der Technik identifizierten relativ aufwendigen Überwachungs- und Regelungssysteme kann es als vorrangiges Ziel der vorliegenden Erfindung betrachtet werden, eine vergleichsweise einfach aufgebaute und mit wenig Aufwand zu betreibende Einrichtung zur Verfügung zu stellen, welche die Mischungsgüte und Anzeichen einer Schwimmschichtbildung erfasst, dies den Anlagenbetreibern übermittelt und die Rührung des Substrats entsprechend dem Bedarf angleicht.

Dieses Ziel wird mit der Einrichtung des unabhängigen Anspruchs 1 sowie mit einem Verfahren mit den Merkmalen des unabhängigen Verfahrensanspruchs erreicht. Merkmale vorteilhafter Weiterbildungen der Erfindung finden sich in den abhängigen Ansprüchen.

Zur Erreichung des genannten Ziels schlägt die Erfindung eine Einrichtung zur Erfassung mindestens eines Zustandsparameters eines gärfähigen Materials oder Substrats in einem Reaktorbehälter vor, in welchem mindestens ein Aktor Substratumwälzungen herbeiführt, wobei mindestens eine unterhalb eines Gärsubstratpegels angeordnete Sensor- und/oder Kraftmessvorrichtung Bewegungskräfte des Gärsubstrats ermittelt, welche durch die Substratumwälzung auf die Kraftmessvorrichtung einwirken und welche Sensor- und/oder Kraftmessvorrichtung mit mindestens einer Datenübertragungseinheit verbunden ist, welche Antriebsparameter des Aktors berücksichtigt und auf Basis der ermittelten Bewegungskräfte einen Zustandsparameter oder ggf. auch mehrere Zustandsparameter des Gärsubstrats ermittelt und/oder berechnet. Als Antriebsparameter des Aktors kommen insbesondere dessen Drehzahl und/oder Stromverbrauch in Frage, da ein solcher Aktor insbesondere durch ein elektromotorisch angetriebenes Rührwerk gebildet sein kann, dessen Drehzahl steuerbar ist, und dessen Stromverbrauch mit der Drehzahl sowie den Fließeigenschaften des damit umgewälzten Substrats veränderlich ist.

Die genannte erfindungsmäßige Einrichtung zur Erfassung eines Zustandsparameters von Gärsubstrat in einem mindestens teilweise mit gärfähigem Material beladenen oder beladbaren Reaktorbehälter umfasst dabei zumindest folgende Elemente: Zur Erfassung der Bewegungskräfte des Gärsubstrats durch die Substratumwälzung, wird mindestens eine geeignete Sensoreinrichtung und/oder Kraftmesseinrichtung unterhalb des Gärsubstratpegels in einem Reaktorbehälter angeordnet.

Die solchermaßen erfassten Daten der mindestens einen Sensor- und/oder Kraftmessvorrichtung und/oder die Antriebsparameter des Aktors wie etwa eine Drehzahl und/oder der Stromverbrauch des Aktors werden an mindestens eine Datenübertragungseinrichtung (Gateway) übermittelt.

Die an die Datenübertragungseinrichtung übermittelten Daten können wahlweise durch eine Internetverbindung an eine Daten-Cloud gesendet werden, welche die Daten verarbeitet und den Anlagenbetreibern prozessoptimierende Steuerungseingriffe in den Gärprozess und Kontrollfunktionen ermöglicht.

Gemäß einer Variante der erfindungsmäßigen Einrichtung kann die Kraftmessvorrichtung unterhalb des Substratpegels an einer im Fermenter geeigneten Position zur Ermittlung der Bewegungskräfte des Gärsubstrats eingerichtet werden. Gemäß einer alternativen Ausführungsvariante der Erfindung kann der die im Reaktorbehälter angeordnete Sensoreinrichtung jedoch auch durch einen Ultraschallsensor gebildet sein, der die Bewegungsparameter des flüssigen oder halbflüssigen Substrats erfassen und als elektrische Signalwerte an die Datenübertragungseinrichtung übermitteln kann.

Bei den ermittelten Zustandsparametern kann es sich insbesondere um eine Umwälz- und/oder Fließgeschwindigkeit des Gärsubstrats innerhalb des Reaktorbehälters handeln, da diese Umwälz- und/oder Fließgeschwindigkeit einen guten Indikator für den Zustand des Gärsubstrats bildet. Je zäher die Flüssigkeit, desto geringer wird die Umwälzgeschwindigkeit sein, und desto eher und/oder in kürzeren Zeitintervallen sollte das Substrat umgewälzt werden. Über die Konsistenz des Substrats kann demzufolge eine Kraftmessvorrichtung Auskunft geben, gebildet bspw. durch mindestens eine Wäge-Zelle, die bei dünnflüssigerem Substrat eine geringere Krafteinwirkung bei gegebener Umwälzgeschwindigkeit erfährt als bei zäherem Substratzustand.

Wahlweise kann die Sensoreinrichtung auch durch mindestens einen innerhalb des Reaktorbehälters angeordneten Ultraschallsensor gebildet sein. Auch ein solcher Sensor liefert aussagekräftige Werte, die vom Substratzustand abhängen. Bei den Bewegungen und/oder Bewegungskräften handelt es sich vorzugsweise um die durch den mindestens einen Aktor erzeugten Fließgeschwindigkeiten des Gärsubstrats.

Eine Datenverarbeitungseinrichtung kann dafür hergerichtet sein, um die Antriebsparameter des mindestens einen Aktors und/oder die erzeugte Strömungsbewegung zu erfassen. Außerdem kann die Datenverarbeitungseinrichtung bspw. den durch mindestens einen Aktor beanspruchten Stromverbrauch und/oder die Drehzahl, und/oder die erzeugte Strömungsbewegung erfassen. Auch kann vorgesehen sein, dass durch die Datenübertragungseinrichtung die Daten an eine Daten-Cloud übermittelbar sind und zur Ermittlung der Fließgeschwindigkeit des Gärsubstrats verarbeitet werden. Eine besonders vorteilhafte Variante der erfindungsgemäßen Einrichtung sieht vor, dass auf Grundlage der ermittelten Daten eine Steuerungs- und Regelungsfunktion zur Regelung der Betriebszeiten des Aktors errechnet und/oder definiert wird.

Die Einrichtung kann sich in einem Reaktorbehälter befinden, der eine durch Beton, Stahlbeton oder Stahl gebildete Wandung aufweist, und der zumindest teilweise mit gärfähigem Material oder Substrat beladen oder beladbar ist. Ein solcher Reaktorbehälter mit einer Einrichtung wie oben beschrieben kann Teil einer Biogasanlage sein.

Zur Erreichung des oben genannten Ziels schlägt die Erfindung weiterhin ein Verfahren zur Erfassung mindestens eines Zustandsparameters eines gärfähigen Materials oder Substrats in einem Reaktorbehälter mit den Merkmalen des unabhängigen Verfahrensanspruchs vor. In dem Reaktorbehälter sorgt mindestens ein Aktor für Umwälzungen des Substrats. Mindestens eine unterhalb eines Gärsubstratpegels angeordnete Sensor- und/oder Kraftmessvorrichtung ermittelt Bewegungen und/oder Bewegungskräfte des Gärsubstrats, welche durch die Substratumwälzung auf die Sensor- und/oder Kraftmessvorrichtung einwirken. Die Sensor- und/oder Kraftmessvorrichtung ist mit mindestens einer Datenübertragungseinrichtung verbunden, welche aus Antriebsparametern des mindestens einen Aktors sowie auf Basis der ermittelten Bewegungskräfte einen Zustandsparameter oder mehrere Zustandsparameter des Gärsubstrats ermittelt. Bei wenigstens einem Zustandsparameter kann es sich insbesondere um eine Umwälz- und/oder Fließgeschwindigkeit des Gärsubstrats innerhalb des Reaktorbehälters handeln. Zudem kann der wenigstens eine Zustandsparameter mittels einer Kraftmessvorrichtung und/oder mittels einer Wäge-Zelle und/oder mittels eines Ultraschallsensors erfasst werden. Zudem kann es sich bei den Bewegungen und/oder Bewegungskräften um die durch den mindestens einen Aktor erzeugten Fließgeschwindigkeiten des Gärsubstrats handeln.

In einer bevorzugten Ausführungsform des erfindungsgemäßen Verfahrens wird die Kraftmessvorrichtung oder der Ultraschallsensor in einem mit Gärsubstrat beladenen Reaktorbehälter unterhalb einer Gasphase und/oder unterhalb eines Flüssigkeitsspiegels (Substratpegels) angeordnet. Üblicherweise sind derartige Reaktorbehälter mit einer Folienhaube versehen, unterhalb welcher sich bei einer Gärreaktion des Gärsubstrats entstehendes oder entstandenes Gas sammelt. Bei dem genannten Gas kann es sich insbesondere um Methangas (CH₄) handeln, welches ggf. bei Kontakt mit Luft ein explosives Gemisch bilden kann. Zum Schutz vor einer möglichen Explosion aufgrund eines entzündbaren Methan-Luft-Gemisches unterliegen die Reaktorbehälter üblicherweise strengen Explosionsschutzauflagen. Folglich werden an die mindestens eine Kraftmessvorrichtung oder den Ultraschallsensor sowie die elektrischen Leitungen und Anschlüsse spezielle explosionsschutztechnische Voraussetzungen gestellt, um ggf. bei Kontakt mit dem entzündbaren Methan-Luft-Gemisch eine Entzündung auszuschließen. Beispielsweise kann die Einrichtung des Sensormoduls durch die Gasphase des Fermenters in das Substrat erfolgen. Außerdem besteht die Möglichkeit, die elektrische Leitung der Kraftmessvorrichtung und/oder des Ultraschallsensors direkt durch die Fermenterwandung des Biogasreaktors unterhalb des Substratpegels einzurichten. Folglich bleibt die Gasphase von den Leitungen unberührt und die Explosionsgefahr kann minimiert oder sogar eliminiert werden. Die Anordnung des Sensormoduls unterhalb des Substratpegels kann auch bei nicht entleertem Reaktorbehälter erfolgen, wohingegen eine Installation des Sensormoduls über die Gasphase aus explosionsschutztechnischen Gründen sehr wahrscheinlich eine Entleerung des Fermenters erforderlich machen wird, was mit zusätzlichen finanziellen Aufwendungen für die Anlagenbetreiber verbunden ist. In beiden Fällen werden erhöhte sicherheitstechnische Maßnahmen zur Gewährleistung der Dichtigkeit ggf. gegen Gasaustritt bzw. gegen Substratsaustritt erforderlich.

Wie bereits erwähnt, ist die mindestens eine Kraftmessvorrichtung zur Erfassung der Substratbewegungen des im Reaktorbehälter befindlichen gärfähigen Materials oder Substrats vorgesehen und/oder ausgebildet. Für das Sensormodul ist die wenigstens eine Kraftmessvorrichtung des Sensormoduls hierbei insbesondere in Form einer Wäge-Zelle gebildet.

Wie oben zudem erwähnt, ist die mindestens eine - alternativ einsetzbare oder auch zusätzlich zur vorhandenen Kraftmessvorrichtung einsetzbare - Sensoreinrichtung zur Erfassung der Substratbewegungen des im Reaktorbehälter befindlichen gärfähigen Materials oder Substrats bspw. durch einen Ultraschallsensor gebildet. Für das Sensormodul ist der wenigstens eine Ultraschallsensor des Sensormoduls hierbei insbesondere in Form eines Halbleitersensors o. dgl. gebildet.

In einer bevorzugten Ausführungsform können in einem außerhalb des Fermenters reichenden Abschnitt elektrische Anschlüsse zur Übermittlung der elektrischen Sensorsignale der mindestens einen Kraftmessvorrichtung und/oder des Ultraschallsensors des Sensormoduls vorgesehen sein. Zudem kann die mindestens eine Kraftmessvorrichtung und/oder der mindestens eine Ultraschallsensor den Zustandsparameter des im Erfassungsbereich des Sensormoduls befindlichen Innenraumes des Reaktorbehälters, insbesondere seiner Materialfüllung und/oder der darin befindlichen Flüssigkeit in elektrische Signale wandeln, die mittels der Datenübertragungseinrichtung erfasst werden.

Die so erfassten Daten können an einen Cloud-Speicher übertragen werden. Auf diese Weise ist es nicht notwendig, lange Datenleitungen ausgehend von der Datenübertragungseinrichtung zu verlegen, sondern es wird zur Datenübertragung eine Internetanbindung genutzt. Dies ermöglicht den Anlagenbetreibern auf den Cloud-Speicher von unterschiedlichen Orten auf die ermittelten Daten zuzugreifen. Folglich können die Anlagenbetreiber an der Biogasanlage Kontroll- sowie Steuerungsfunktionen zur Optimierung des mikrobiellen Wachstums vornehmen. Dies ermöglicht den Betreibern eine flexiblere Kontrolle und ggf. einen schnelleren Eingriff in den Vergasungsprozess durch den Zugriff der Daten über den Cloud-Speicher, ohne unmittelbar vor Ort sein zu müssen. Außerdem können durch die erfassten Parameter Rückschlüsse auf das zeitliche Strömungsverhalten des Gärsubstrats nach erfolgter Rührung durch den Aktor gezogen und die Laufzeiten des Aktors optimiert werden. Dies hat zum Ziel, den Energieaufwand für den Aktor zu minimieren und somit Kosten einzusparen, ohne den Vergärungsprozess und damit die Gasbildungsrate negativ zu beeinflussen.

Ergänzend können mittels geeigneter Sensoren auch Temperaturen im Gärsubstrat gemessen und der Datenverarbeitungs- und/oder Datenübertragungseinrichtung zur Verfügung gestellt werden, die somit weitere Parameter verfügbar hat, um präzisere Rückschlüsse auf den Zustand des Gärsubstrats und seine Konsistenz ziehen zu können. Ebenso kann der verwendete Ultraschallsensor Rückschlüsse auf den sog. TS-Gehalt des Gärsubstrats (Gehalt an Trockensubstanz, z.B. etwa 10%) erlauben. Weitere Informationen lassen sich bspw. über den Einsatz einer Kamera gewinnen, die oberhalb der Gasphase angeordnet sein kann, und die Bilder aus dem Reaktorbehälter liefern kann, die dem Anlagenbetreiber weitere Informationen über den Zustand des im Behälter befindlichen Substrats liefern können. Alle diese genannten Werte bzw. Sensorwerte und Bildinformationen können für sich oder in kombinierter Form zur Überwachung und Steuerung der Gärprozesse im Reaktorbehälter verwendet und eingesetzt werden.

Es sei an dieser Stelle ausdrücklich klargestellt, dass alle Aspekte, die im Zusammenhang mit der erfindungsgemäßen Einrichtung beschrieben wurden, gleichermaßen für den Reaktorbehälter, der eine durch Beton, Stahlbeton oder Stahl gebildete Wandung aufweist, und der mindestens teilweise mit gärfähigem Material oder Substrat beladen oder beladbar ist, gelten können, und mit einer Kraftmessvorrichtung nach vorliegender erfindungsgemäßen Einrichtung ausgestattet wurde. Gleiches gilt für die mit einem solchen Reaktorbehälter oder mit mehreren solchen Reaktorbehältern ausgestattete Biogasanlage. Alle genannten Aspekte und Merkmale können daher gleichermaßen auf einen Reaktorbehälter und/oder eine Biogasanlage mit mindestens einem Reaktorbehälter gelesen und verstanden werden.

Die nachfolgenden Ausführungen fassen nochmal einige Aspekte der zuvor bereits in verschiedenen Ausführungsvarianten erläuterten Erfindung zusammen, konkretisieren einige Aspekte, sollen jedoch nicht im Widerspruch zu den bereits gemachten Ausführungen gesehen werden, sondern in Zusammenschau, bei Zweifeln ggf. als speziellere Ausführungsvarianten und/oder Abwandlungen. So kann, wie bereits oben mehrfach erwähnt, die erfindungsgemäße Einrichtung zumindest einen Aktor umfassen. Zudem umfasst die Einrichtung wenigstens eine unterhalb eines Gärsubstratpegels angeordnete oder einzurichtende Kraftmessvorrichtung, welche im Erfassungsbereich eines Sensors die durch die Rührung des Aktors begründeten Bewegungskräfte eines Gärsubstrats ermittelt. Wahlweise oder zusätzlich kann die Einrichtung einen Ultraschallsensor aufweisen, der im Erfassungsbereich des Sensors die durch die Rührung des Aktors begründeten Bewegungskräfte des Gärsubstrats ermittelt. Ein Sensormodul kann die Kraftmessvorrichtung und/oder den Ultraschallsensor sowie elektrische Leitungen und elektrische Anschlüsse aufnehmen oder enthalten. Von den Sensoren werden elektrische Sensorsignale geliefert, die Daten in Gestalt von elektrischen Parametern des Aktors wie Rührerdrehzahl und Stromverbrauch liefern. Eine Datenübertragungseinrichtung erfasst die übermittelten elektrischen Sensorsignale und elektrischen Parameter des Aktors. Eine optionale Daten-Cloud verarbeitet die über eine Internetverbindung gesendeten Daten und Parameter der Datenübertragungseinrichtung und stellt diese bspw. für die Anlagenbetreiber zur Verfügung.

Eine bevorzugte Ausführungsform der erfindungsmäßigen Einrichtung ist in einer Weise ausgestaltet, dass der mindestens eine Sensor durch eine Wandung eines Reaktorbehälters hindurchgeführt ist. Der Reaktorbehälter ist vorzugsweise Teil einer Biogasanlage. Die Wandung des Reaktorbehälters kann hierbei beispielsweise durch Beton, Stahlbeton oder Stahl ausgebildet sein. Bei Anordnung der erfindungsgemäßen Einrichtung kann der Reaktorbehälter insbesondere zumindest teilweise mit gärfähigem Material oder Substrat, kurz Gärsubstrat, gefüllt bzw. beladen sein. Der Reaktorbehälter kann beispielhaft bis zu einer definierten Höhe mit Gärsubstrat beladen sein. Weiterhin ist der Reaktorbehälter z.B. mit einer Folienhaube versehen. Unterhalb der Folienhaube wird bei der Gärreaktion des Gärsubstrats entstehendes oder entstandenes Gas, beispielsweise Methangas (CH₄), gespeichert. Die Rührung und Umwälzung des Gärsubstrats erfolgt durch einen Aktor, welcher unterhalb des Gärsubstratpegels angeordnet ist, wobei der Aktor bspw. durch ein Rührwerk oder durch mehrere Rührwerke gebildet sein kann.

Gemäß der vorliegenden Einrichtung soll nun eine Kraftmessvorrichtung zur Ermittlung von Bewegungskräften des Gärsubstrats, welche durch die Substratumwälzung auf die Kraftmessvorrichtung und/oder auf den Ultraschallsensor einwirken, in den mit Gärsubstrat gefüllten Reaktorbehälter eingerichtet werden. Als Kraftmessvorrichtung kommt bevorzugt eine Wäge-Zelle zum Einsatz. Wahlweise oder zusätzlich kann ein Ultraschallsensor zur Erfassung der Substratumwälzungen zum Einsatz kommen.

Dazu wird das Sensormodul mit der Kraftmessvorrichtung und/oder dem Ultraschallsensor über die Wandung unterhalb des Gärsubstratpegels in das Gärsubstrat eingebracht. Eine vorgesehene Einbauposition der Kraftmessvorrichtung befindet sich unterhalb eines Gasspiegels. Bei der Wahl der Einbauposition der Kraftmesseinrichtung ist es wichtig, eine Position unterhalb des Gasspiegels bzw. unterhalb des Gärsubstratpegels des Gärsubstrats zu wählen, um außerhalb der Explosionszone des Gases zu bleiben. Hierdurch kann die Explosionsgefahr bei der Installation oder auch beim Betrieb der Kraftmessvorrichtung oder des Ultraschallsensors minimiert oder sogar eliminiert werden. Des Weiteren kann die Installation der Kraftmessvorrichtung und/oder des Ultraschallsensors unterhalb des Gärsubstratpegels auch bei nicht entleertem Reaktorbehälter erfolgen. Die Kraftmessvorrichtung und/oder der Ultraschallsensor ist/sind im Gärsubstrat so zu positionieren, dass der Zustandsparameter korrekt erfasst werden kann. Die elektrischen Sensorsignale werden über die elektrischen Leitungen und elektrischen Anschlüsse an die Datenübertragungseinrichtung geleitet und zusammen mit den Parametern des Aktors erfasst.

Bei einer weiteren Ausführungsform der erfindungsmäßigen Einrichtung, die mit mindestens einer Kraftmessvorrichtung und/oder einem Ultraschallsensor ausgestattet ist, der/die durch die Wandung des Reaktorbehälters reichen, werden die erfassten Daten der Datenübertragungseinrichtung via Internetverbindung an eine Daten-Cloud übermittelt. In der Daten-Cloud werden die erfassten Daten der Kraftmessvorrichtung und/oder des Ultraschallsensors und/oder die Drehzahl und/oder der Stromverbrauch des Aktors verarbeitet bzw. zwischengespeichert. Dies ermöglicht den Anlagenbetreibern durch die Eigenbewegung des Gärsubstrats nach dem Rührvorgang die optimalen Rührintervalle des Aktors zu ermitteln und Schwimmschichten zu vermeiden. Folglich können die Biogasausbeute durch eine optimierte Substratumwälzung gesteigert und steuernde Eingriffe und Überprüfungen der Biogasanlage vorgenommen werden.

Wahlweise kann ein Sensormodul mit der Kraftmessvorrichtung und/oder dem Ultraschallsensor über eine Folienhaube in das Gärsubstrat geführt sein oder werden. Die Einbauposition der Kraftmesseinrichtung oder des Ultraschallsensors über die Gasphase unter den Gärsubstratpegel wird aus explosionsschutztechnischen Gründen eine Entleerung des Gärsubstrats aus dem Innenraum des Reaktorbehälters erforderlich machen. Die elektrischen Sensorsignale werden über die elektrischen Leitungen und elektrischen Anschlüsse zusammen mit den elektrischen Parametern des Aktors an die Datenübertragungseinrichtung geleitet und von dieser erfasst. Die erfassten Daten der Datenübertragungseinrichtung können insbesondere via Internetverbindung an die Daten-Cloud übermittelt werden. In der Daten-Cloud werden die eingehenden Daten verarbeitet und ermöglichen auf diese Weise steuernde Eingriffe und eine Kontrolle und Fernüberwachung der Biogasanlage.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Fig. 1 zeigt eine schematische Abbildung der zur erfindungsgemäßen Einrichtung integrierten bzw. beteiligten Bauteile.
Fig. 2 zeigt eine schematische Abbildung einer bevorzugten Ausführungsform der erfindungsgemäßen Einrichtung.
Fig. 3 zeigt eine weitere schematische Abbildung einer bevorzugten Ausführungsform der erfindungsgemäßen Einrichtung.
Fig. 4 zeigt eine weitere schematische Abbildung einer bevorzugten Ausführungsform der erfindungsgemäßen Einrichtung.
Fig. 5 zeigt eine weitere schematische Abbildung einer bevorzugten Ausführungsform der erfindungsgemäßen Einrichtung.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellte Ausführungsform stellt lediglich ein Beispiel dar, wie das erfindungsgemäße Verfahren durchgeführt werden kann, soll jedoch nicht als abschließende Begrenzung aufgefasst werden.

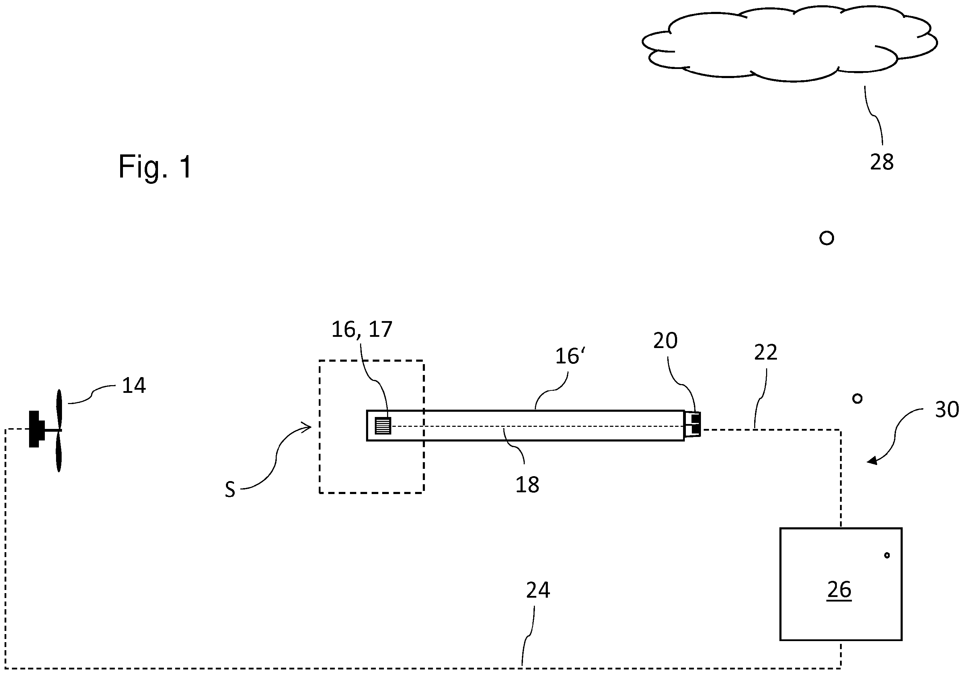

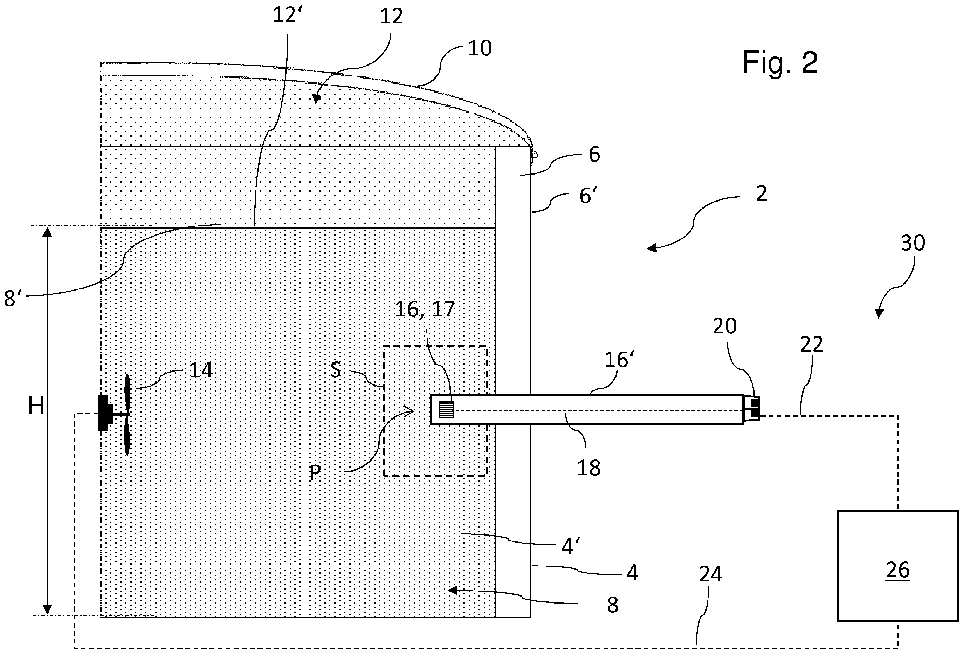

Die schematische Darstellung der Fig. 1 zeigt eine Ausführungsform der erfindungsmäßigen Einrichtung 30 einschließlich eines beteiligten Aktors 14. Die Einrichtung 30 umfasst dabei
- eine unter einem Gärsubstratpegel 8' (vgl. beispielsweise Fig. 2) einzurichtende Kraftmessvorrichtung 16, welche im Erfassungsbereich eines Sensors S die durch die Rührung des Aktors 14 begründeten Bewegungskräfte eines Gärsubstrats 8 ermittelt,
- oder wahlweise einen Ultraschallsensor 17, der im Erfassungsbereich des Sensors S die durch die Rührung des Aktors 14 begründeten Bewegungskräfte des Gärsubstrats 8 ermittelt,
- ein Sensormodul 16', in welchem die Kraftmessvorrichtung 16 und/oder der Ultraschallsensor 17 sowie elektrische Leitungen 18 und elektrische Anschlüsse 20 eingerichtet sind,
- elektrische Sensorsignale 22 und elektrische Parameter 24 des Aktors 14 wie Rührerdrehzahl und Stromverbrauch,
- eine Datenübertragungseinrichtung 26, welche die übermittelten elektrischen Sensorsignale 22 und elektrischen Parameter 24 des Aktors 14 erfasst,
- eine Daten-Cloud 28, welche die über eine Internetverbindung gesendeten Daten und Parameter der Datenübertragungseinrichtung 28 verarbeitet und für die Anlagenbetreiber zur Verfügung stellt.

Die schematische Teilschnittdarstellung der Fig. 2 zeigt eine bevorzugte Ausführungsform der erfindungsmäßigen Einrichtung 30 mindestens eines Sensors 16 durch eine Wandung 6 eines Reaktorbehälters 4. Der Reaktorbehälter 4 ist hierbei vorzugsweise Teil einer Biogasanlage 2. Die Wandung 6 des Reaktorbehälters 4 kann hierbei beispielsweise durch Beton, Stahlbeton oder Stahl ausgebildet sein. Bei Anordnung der erfindungsgemäßen Einrichtung 30 kann der Reaktorbehälter 4 insbesondere zumindest teilweise mit gärfähigem Material 8 oder Substrat 8, kurz Gärsubstrat 8, gefüllt bzw. beladen sein. In der Abbildung der Fig. 2 ist der Reaktorbehälter 4 beispielhaft bis zu einer Höhe H mit Gärsubstrat 8 beladen. Weiterhin ist der Reaktorbehälter 4 mit einer Folienhaube 10 versehen. Unterhalb der Folienhaube 10 wird bei der Gärreaktion des Gärsubstrats 8 entstehendes oder entstandenes Gas 12, beispielsweise Methangas (CH₄), gespeichert. Die Rührung des Gärsubstrats 8 erfolgt durch den schematisch dargestellten Aktor 14, welcher unterhalb des Gärsubstratpegels 8' angeordnet ist.

Gemäß der vorliegenden Einrichtung 30 soll nun eine Kraftmessvorrichtung 16 zur Ermittlung von Bewegungskräften des Gärsubstrats, welche durch die Substratumwälzung auf die Kraftmessvorrichtung 16 und/oder auf den Ultraschallsensor 17 einwirken, in den mit Gärsubstrat 8 gefüllten Reaktorbehälter 4 eingerichtet werden. Als Kraftmessvorrichtung 16 kommt bevorzugt eine Wäge-Zelle zum Einsatz. Wahlweise oder zusätzlich kann ein Ultraschallsensor 17 zur Erfassung der Substratumwälzungen zum Einsatz kommen.

Dazu wird das Sensormodul 16' mit der Kraftmessvorrichtung 16 und/oder dem Ultraschallsensor 17 über die Wandung 6 unterhalb des Gärsubstratpegels 8' in das Gärsubstrat 8 eingebracht. Eine vorgesehene Einbauposition der Kraftmessvorrichtung P befindet sich unterhalb eines Gasspiegels 12'. Bei der Wahl der Einbauposition der Kraftmesseinrichtung P ist es wichtig, eine Position unterhalb des Gasspiegels 12' bzw. unterhalb des Gärsubstratpegels 8' des Gärsubstrats 8 zu wählen, um außerhalb der Explosionszone des Gases 12 zu bleiben. Hierdurch kann die Explosionsgefahr bei der Installation oder auch beim Betrieb der Kraftmessvorrichtung 16 oder des Ultraschallsensors 17 minimiert oder sogar eliminiert werden. Des Weiteren kann die Installation der Kraftmessvorrichtung 16 und/oder des Ultraschallsensors 17 unterhalb des Gärsubstratpegels 8' auch bei nicht entleertem Reaktorbehälter 4 erfolgen. Die Kraftmessvorrichtung 16 und/oder der Ultraschallsensor 17 ist im Gärsubstrat 8 so zu positionieren, dass der Zustandsparameter korrekt erfasst werden kann. Die elektrischen Sensorsignale 22 werden über die elektrischen Leitungen 18 und elektrischen Anschlüsse 20 an die Datenübertragungseinrichtung 26 geleitet und zusammen mit den Parametern des Aktors 14 erfasst.

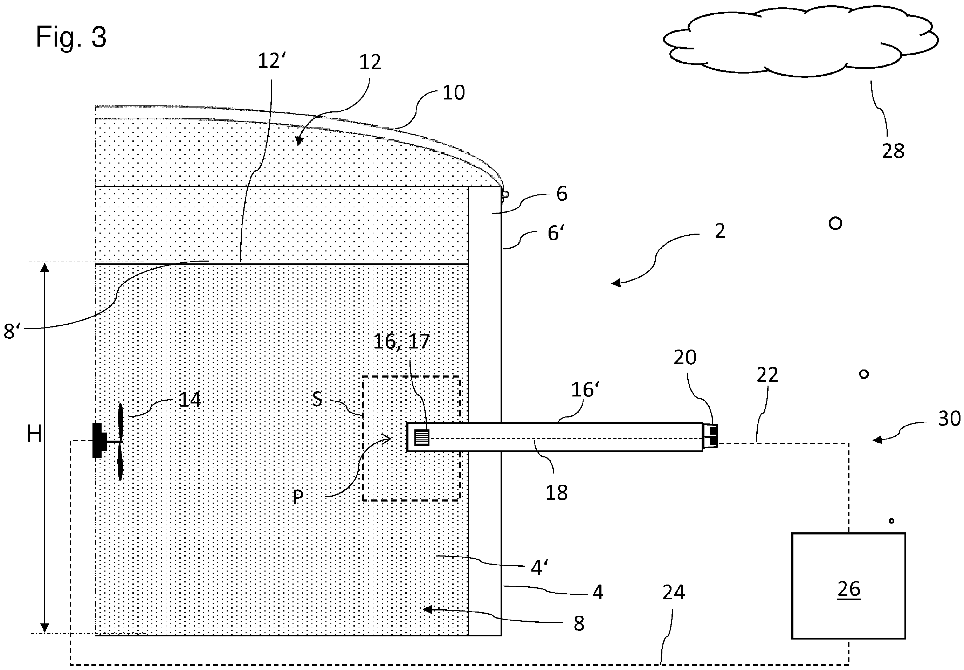

Die schematische Teilschnittdarstellung der Fig. 3 zeigt eine weitere bevorzugte Ausführungsform der erfindungsmäßigen Einrichtung 30 mit mindestens einer Kraftmessvorrichtung 16 oder einem Ultraschallsensor 17, der/die durch die Wandung 6 des Reaktorbehälters 4 reichen. Die erfassten Daten der Datenübertragungseinrichtung 26 werden via Internetverbindung an die Daten-Cloud 28 übermittelt. In der Daten-Cloud 28 werden die erfassten Daten der Kraftmessvorrichtung 16 und/oder des Ultraschallsensors 17 und/oder die Drehzahl und/oder der Stromverbrauch des Aktors 14 verarbeitet. Dies ermöglicht den Anlagenbetreibern durch die Eigenbewegung des Gärsubstrats 8 nach dem Rührvorgang die optimalen Rührintervalle des Aktors 14 zu ermitteln und Schwimmschichten zu vermeiden. Folglich können die Biogasausbeute durch eine optimierte Substratumwälzung gesteigert und steuernde Eingriffe und Überprüfungen der Biogasanlage 2 vorgenommen werden.

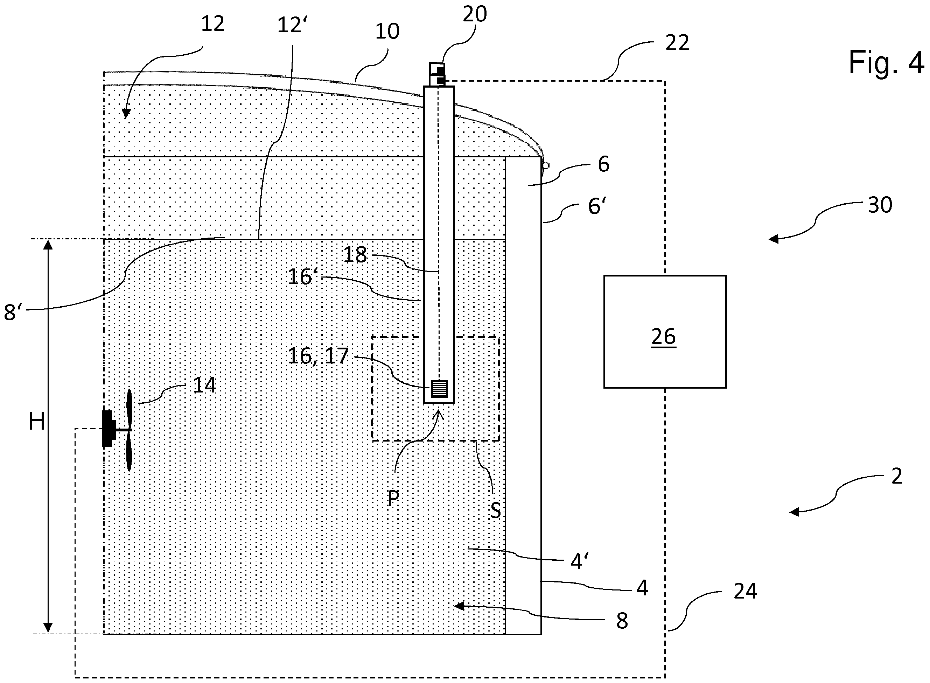

Die schematische Teilschnittdarstellung der Fig. 4 zeigt eine weitere Ausführungsform der erfindungsmäßigen Einrichtung 30 mindestens einer Kraftmessvorrichtung 16 bzw. des mindestens einen Ultraschallsensors 17 über die Gasphase 12' des Reaktorbehälters 4 in das Gärsubstrat 8. Dabei wird das Sensormodul 16' mit der Kraftmessvorrichtung 16 und/oder dem Ultraschallsensor 17 über eine Folienhaube 10 in das Gärsubstrat 8 geführt. Die Einbauposition P der Kraftmesseinrichtung 16 oder des Ultraschallsensors 17 über die Gasphase 12' unter den Gärsubstratpegel 8' wird aus explosionsschutztechnischen Gründen eine Entleerung des Gärsubstrats 8 aus dem Innenraum des Reaktorbehälters 4' erforderlich machen. Die elektrischen Sensorsignale 22 werden über die elektrischen Leitungen 18 und elektrischen Anschlüsse 20 zusammen mit den elektrischen Parametern 24 des Aktors 14 an die Datenübertragungseinrichtung 26 geleitet und von dieser erfasst.

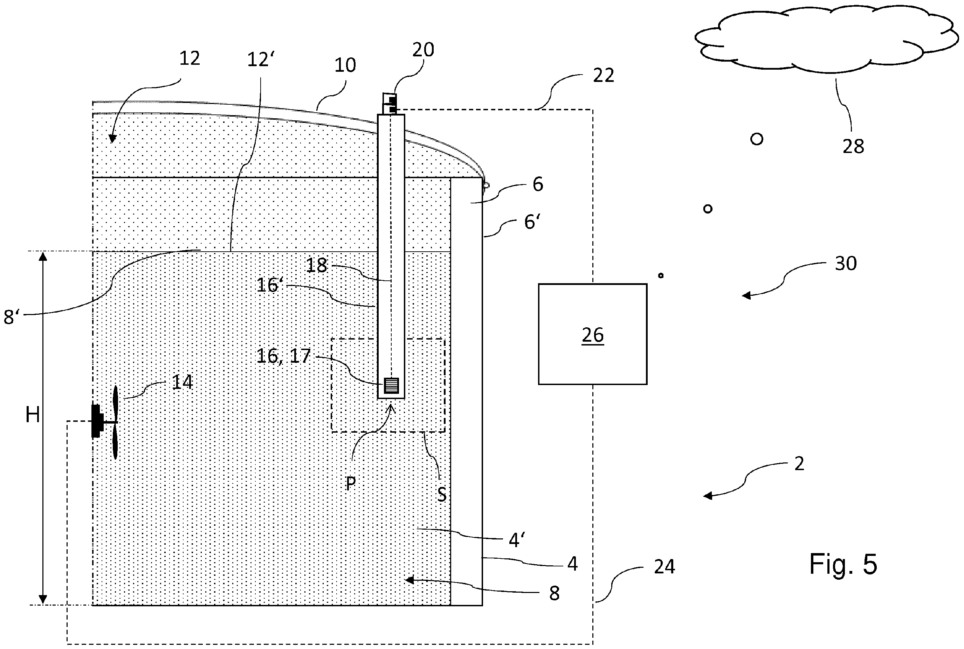

Die schematische Teilschnittdarstellung der Fig. 5 zeigt eine weitere bevorzugte Ausführungsform der erfindungsmäßigen Einrichtung 30 mindestens einer Kraftmessvorrichtung 16 oder eines Ultraschallsensors 17 durch die Gasphase 12' des Reaktorbehälters 4 in das Gärsubstrat 8. Die erfassten Daten der Datenübertragungseinrichtung 26 werden via Internetverbindung an die Daten-Cloud 28 übermittelt. In der Daten-Cloud 28 werden die eingehenden Daten verarbeitet und ermöglichen steuernde Eingriffe und Kontrolle der Biogasanlage.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

2 Biogasanlage
4 Reaktorbehälter
4' Innenraum des Reaktorbehälters
6 Wandung
6' Außenseite der Wandung
8 Material, Substrat, Gärsubstrat
8' Gärsubstratpegel
10 Folienhaube
12 Gas
12' Gasspiegel, Gasphase
14 Aktor
16 Kraftmessvorrichtung, Sensor
16' Sensormodul
17 Ultraschallsensor
18 elektrische Leitungen
20 elektrische Anschlüsse
22 elektrische Sensorsignale
24 elektrische Parameter
26 Datenübertragungseinrichtung, Gateway
28 Daten-Cloud
30 Einrichtung

H Füllhöhe des Gärsubstrats im Reaktorbehälter
P Einbauposition der Kraftmessvorrichtung / des Ultraschallsensors
S Erfassungsbereich der Kraftmesseinrichtung / des Ultraschallsensors bzw. des Sensormoduls

## Patentansprüche

1. Einrichtung (30) zur Erfassung mindestens eines Zustandsparameters eines gärfähigen Materials (8) oder Substrats (8) in einem Reaktorbehälter (4), der mit mindestens einem Aktor (14) zur Substratumwälzung ausgestattet ist oder in welchem mindestens ein Aktor (14) Substratumwälzungen herbeiführt,
- wobei mindestens eine unterhalb eines Gärsubstratpegels (8') des gärfähigen Materials oder Substrats (8) angeordnete Sensor- und/oder Kraftmessvorrichtung (16) Bewegungen und/oder Bewegungskräfte des Gärsubstrats (8) ermittelt, welche durch die Substratumwälzung auf die Sensor- und/oder Kraftmessvorrichtung (16) einwirken,
- welche Sensor- und/oder Kraftmessvorrichtung (16) mit mindestens einer Datenübertragungseinrichtung (26) verbunden ist,
welche aus Antriebsparametern des mindestens einen Aktors (14) sowie auf Basis der ermittelten Bewegungskräfte wenigstens einen Zustandsparameter des Gärsubstrats (8) ermittelt.

2. Einrichtung (30) nach Anspruch 1, wobei es sich bei dem wenigstens einen Zustandsparameter um eine Umwälz- und/oder Fließgeschwindigkeit des Gärsubstrats (8) innerhalb des Reaktorbehälters (4) handelt.

3. Einrichtung (30) nach Anspruch 1 oder 2, bei der die Kraftmessvorrichtung (16) durch mindestens eine Wäge-Zelle gebildet ist oder eine solche Wäge-Zelle umfasst.

4. Einrichtung (30) nach Anspruch 1 oder 2, bei der die Sensoreinrichtung (17) durch mindestens einen innerhalb des Reaktorbehälters (4) angeordneten Ultraschallsensor (17) gebildet ist oder einen solchen Ultraschallsensor (17) umfasst.

5. Einrichtung (30) nach einem der Ansprüche 1 bis 4, wobei es sich bei den Bewegungen und/oder Bewegungskräften um die durch den mindestens einen Aktor (14) erzeugten Fließgeschwindigkeiten des Gärsubstrats (8) handelt.

6. Einrichtung (30) nach einem der Ansprüche 1 bis 5, wobei eine Datenverarbeitungseinrichtung (26) die Antriebsparameter des mindestens einen Aktors (14) und/oder die erzeugte Strömungsbewegung erfasst.

7. Einrichtung (30) nach Anspruch 6, wobei die Datenverarbeitungseinrichtung (26) den durch mindestens einen Aktor (14) beanspruchten Stromverbrauch, und/oder die Drehzahl, und/oder die erzeugte Strömungsbewegung erfasst.

8. Einrichtung (30) nach Anspruch 6 oder 7, wobei durch die Datenübertragungseinrichtung (26) die Daten an eine Daten-Cloud (28) übermittelbar sind und zur Ermittlung der Fließgeschwindigkeit des Gärsubstrats verarbeitet werden.

9. Einrichtung nach Anspruch 8, bei der auf Grundlage der ermittelten Daten eine Steuerungs- und Regelungsfunktion zur Regelung der Betriebszeiten des Aktors (14) errechnet und/oder definiert wird.

10. Reaktorbehälter (4), der eine durch Beton, Stahlbeton oder Stahl gebildete Wandung (6) aufweist, und der zumindest teilweise mit gärfähigem Material (8) oder Substrat (8) beladen oder beladbar ist, der mit mindestens einer Einrichtung (30) gemäß einem der Ansprüche 1 bis 9 ausgestattet ist.

11. Biogasanlage (2) mit mindestens einem Reaktorbehälter (4) gemäß Anspruch 10, der mit mindestens einer Einrichtung (30) gemäß einem der Ansprüche 1 bis 9 ausgestattet ist.

12. Verfahren zur Erfassung mindestens eines Zustandsparameters eines gärfähigen Materials (8) oder Substrats (8) in einem Reaktorbehälter (4), in welchem mindestens ein Aktor (14) Substratumwälzungen herbeiführt, wobei mindestens eine unterhalb eines Gärsubstratpegels (8') angeordnete Sensor- und/oder Kraftmessvorrichtung (16) Bewegungen und/oder Bewegungskräfte des Gärsubstrats (8) ermittelt, welche durch die Substratumwälzung auf die Sensor- und/oder Kraftmessvorrichtung (16) einwirken, und welche Sensor- und/oder Kraftmessvorrichtung (16) mit mindestens einer Datenübertragungseinrichtung (26) verbunden ist, welche aus Antriebsparametern des mindestens einen Aktors (14) sowie auf Basis der ermittelten Bewegungskräfte einen Zustandsparameter oder mehrere Zustandsparameter des Gärsubstrats (8) ermittelt.

13. Verfahren nach Anspruch 12, bei dem es sich bei wenigstens einem Zustandsparameter um eine Umwälz- und/oder Fließgeschwindigkeit des Gärsubstrats (8) innerhalb des Reaktorbehälters (4) handelt.

14. Verfahren nach Anspruch 12 oder 13, bei dem der wenigstens eine Zustandsparameter mittels einer Kraftmessvorrichtung (16) und/oder mittels einer Wäge-Zelle und/oder mittels eines Ultraschallsensors (17) erfasst wird.

15. Verfahren nach einem der Ansprüche 12 bis 14, bei dem es sich bei den Bewegungen und/oder Bewegungskräften um die durch den mindestens einen Aktor (14) erzeugten Fließgeschwindigkeiten des Gärsubstrats (8) handelt.
